# EUROPÄISCHE PATENTSCHRIFT

(11) **EP 4 216 880 B1**
(45) Veröffentlichungstag und Bekanntmachung des Hinweises auf die Patenterteilung: **06.08.2025**
(21) Anmeldenummer: 21782930.8
(22) Anmeldetag: 22.09.2021
(51) Int. Cl.: A61F 2/64, A61F 2/66, A61F 2/60, A61F 2/76

(54) **VERFAHREN ZUR STEUERUNG EINES PROTHESENFUSSES**
METHOD FOR CONTROLLING A PROSTHETIC FOOT
PROCÉDÉ DE COMMANDE D'UNE PROTHÈSE DE PIED

(30) Priorität: 28.09.2020 DE 102020125256
(43) Veröffentlichungstag der Anmeldung: 02.08.2023
(73) Patentinhaber: Otto Bock Healthcare Products GmbH, 1110 Wien (AT)
(72) Erfinder: PAPPE, Alexander, 1180 Wien (AT); SEYR, Martin, 1070 Wien (AT); BOHLAND, Andreas, 1120 Wien (AT); HOFMANN, Thomas, 1030 Wien (AT); SEIFERT, Dirk, 1030 Wien (AT); ERNST, Michael, 37181 Hardegsen (DE)
(74) Vertreter: Gramm, Lins & Partner Patent- und Rechtsanwälte PartGmbB
(86) Internationale Anmeldenummer: PCT/EP2021/076059
(87) Internationale Veröffentlichungsnummer: WO 2022/063826

(56) Entgegenhaltungen:
- EP-A1- 2 498 724
- EP-A1- 3 035 895
- EP-B1- 2 498 724
- EP-B1- 3 035 895
- US-A1- 2015 257 902

## Beschreibung

Die Erfindung betrifft ein Verfahren zur Steuerung eines Prothesenfußes, der ein Oberteil mit einem Befestigungselement für eine proximale Prothesenkomponente und ein um eine Schwenkachse schwenkbar daran gelagertes Fußteil aufweist, mit einer Widerstandseinrichtung, mit der einer Verschwenkbewegung des Fußteils relativ zu dem Oberteil ein einstellbarer Widerstand entgegengesetzt wird und mit einer Steuerungseinrichtung, die mit der Widerstandseinrichtung und mit zumindest einem Sensor gekoppelt ist und über die in Abhängigkeit von Sensordaten der Widerstand gegen eine Verschwenkung eingestellt wird.

Prothesenfüße sind Teil einer prothetischen Versorgung und werden entweder unmittelbar an einem Prothesenschaft oder an einem Unterschenkelrohr gelagert. Neben starren Prothesenfüßen mit einem gepolsterten Fersenbereich, sogenannten SACH-Füßen, existieren komplexe Prothesenfüße mit Federanordnungen und Dämpferan-ordnungen innerhalb des Prothesenfußes. Verstelleinrichtungen innerhalb eines Prothesenfußes können vorgesehen sein, um Eigenschaften zu verändern, die das Auftreten und das Abrollen betreffen.

Neben starr an einem Befestigungsabschnitt oder einer Befestigungseinrichtung angeordneten Prothesenfüßen existieren gelenkig an einem Oberteil gelagerte Prothesenfüße mit einem Prothesenfußgelenk, um eine Verschwenkung um in der Regel eine Schwenkachse zu ermöglichen. Zur Beeinflussung des Auftritts- und Abrollverhaltens sind Widerstandseinrichtungen vorgesehen, die auf Basis von Sensordaten verstellt werden können. Die Widerstandseinrichtungen sind beispielsweise Fluiddämpfer mit Ventilen zur Beeinflussung eines Strömungswiderstandes, Widerstandseinrichtungen auf Basis magnetorheologischer Einrichtungen, bei denen über Änderungen in einem Magnetfeld die Viskosität eines Mediums verändert wird, unter anderem Bremseinrichtungen oder Antriebe, die durch eine entsprechende Verschaltung einen Widerstand gegen eine Verschwenkbewegung bereitstellen. Die Verschwenkung erfolgt ausgehend aus einer Ausgangsstellung in Richtung auf den Fußboden mit einer sogenannten Plantarflexion und in umgekehrter Richtung im Rahmen einer Dorsalflexion, bei der die Fußspitze in Richtung auf das Schienbein bewegt wird. Die Verstellung der jeweiligen Widerstände erfolgt auf Grundlage der Auswertung von Sensordaten. Die Auswertung erfolgt in einer Steuerungseinrichtung, die mit den Sensoren gekoppelt ist. Die Steuerungseinrichtung veranlasst geeignete Maßnahmen, um den Widerstand wie gewünscht anzupassen, beispielsweise werden Ventile verstellt, Magnetfelder aufgebaut oder Motore in einen Generatorbetrieb versetzt.

Ein Beispiel eines Prothesenfußes und einer Steuerung ist in der WO 2014/079588 A1 offenbart. Dabei wird über eine Kraftübertragungseinrichtung von einem Oberschenkelteil bei einer Knieflexion eine Plantarflexion eines Fußteils bewirkt. Die Kraftübertragung kann hydraulisch erfolgen, ein Ventilblock wird in Abhängigkeit von einem Kniewinkel und einem Plantarflexionswinkel geschaltet. Lagesensoren oder Kraft- oder Momentensensoren sind vorgesehen, um die Lage im Raum, Kräfte oder Momente zu bestimmen und zur Steuerung der Ventile zu nutzen.

Die EP 2 498 724 B1 betrifft ein Verfahren zur Steuerung eines künstlichen Kniegelenkes mit einer Widerstandseinrichtung, über die der Beugewiderstand oder Streckwiderstand in Abhängigkeit von Sensordaten verändert wird. Der Widerstand in der Standphase oder während des Bestehens wird in Abhängigkeit von der Bodenreaktionskraft bis zu einer Sperre des Kniegelenkes erhöht.

Die EP 3 035 895 B1 betrifft ein Verfahren zur Steuerung eines künstlichen Kniegelenkes mit einer Unterschenkelkomponente und einer Widerstandseinrichtung, über die der Beugewiderstand in Abhängigkeit von Sensordaten verändert wird. Eine lineare Beschleunigung der Unterschenkelkomponente wird ermittelt und mit zumindest einem Schwellwert verglichen, wobei bei Unterschreiten eines Grenzwertes der Linearbeschleunigung der Unterschenkelkomponente der Beugewiderstand verringert wird.

Die US 2015/257902 A1 betrifft unter anderem ein computergesteuertes Knöchelgelenksystem mit einem Fußteil und Befestigungsmitteln zum Festlegen des prothetischen Knöchelgelenkes an einem Patienten, wobei zwischen dem Befestigungsmittel und dem Fußteil ein Dämpfersystem angeordnet ist, das eine Verdrehung des Fußteils in Bezug auf den Nutzer beeinflusst. Das Dämpfersystem kann sowohl als passives Dämpfersystem als auch als aktiv angetriebene Steuerung ausgebildet sein. Auf der Grundlage von Sensorinformationen wird der Widerstand eingestellt. Der Bodenkontakt des Fußteils wird über zumindest einen Sensor ermittelt.

Aufgabe der vorliegenden Erfindung ist es, insbesondere bei Situationsübergängen, beispielsweise zu Beginn des Gehens nach einer Phase des Stehens, eine zuverlässige und sichere Steuerung des Widerstandsverhaltens des Prothesenfußes bereitzustellen.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen, der Beschreibung und den Figuren offenbart.

Das Verfahren zur Steuerung eines Prothesenfußes, der ein Oberteil mit einem Befestigungselement für eine proximale Prothesenkomponente und ein um eine Schwenkachse schwenkbar daran gelagertes Fußteil aufweist, mit einer Widerstandseinrichtung, mit der einer Verschwenkbewegung des Fußteils relativ zu dem Oberteil ein einstellbarer Widerstand entgegengesetzt wird und mit einer Steuerungseinrichtung, die mit der Widerstandseinrichtung und mit zumindest einem Sensor gekoppelt ist und über die in Abhängigkeit von Sensordaten der Widerstand gegen eine Verschwenkung eingestellt wird, sieht vor, dass über zumindest einen Sensor ein Bodenkontakt des Fußteils mit dem Boden ermittelt wird, dass weiterhin die Raumlage des Fußteils und/oder des Oberteils über eine inertiale Messeinheit ermittelt wird und dass der Widerstand gegen eine Verschwenkung in Abhängigkeit von dem Vorliegen oder Nichtvorliegen eines Bodenkontaktes und der ermittelten Raumlage und/oder dem ermittelten Verlauf der Raumlage verändert wird, wobei der Winkel zwischen dem Fußteil und dem Oberteil gemessen und eine Dorsalbewegung in Abhängigkeit von dem gemessenen Winkel zwischen dem Oberteil und dem Fußteil und/oder einem Raumlagewinkel ermöglicht oder unterbunden wird. Aufgrund der gemeinsamen Auswertung sowohl der Raumlage als auch des Vorhandenseins eines Bodenkontaktes ist es möglich, auf der Grundlage der Sensordaten und der daraus rückgeschlossenen Situation des Prothesennutzers eine verbesserte Verstellung der jeweiligen Widerstände in Plantarflexionsrichtung und Dorsalflexionsrichtung zu ermöglichen. Dabei kann sowohl zunächst eine Veränderung allein auf Basis von Bodenkontaktdaten als auch eine Veränderung auf Basis der Raumlagedaten errechnet und eine Überlagerung stattfinden, wobei der jeweils sichere Widerstandswert eingestellt wird. Eine Dorsalbewegung wird in Abhängigkeit von dem gemessenen Winkel zwischen dem Oberteil und dem Fußteil und/oder einem Raumlagewinkel ermöglicht oder unterbunden. Dadurch wird eine Dorsalflexion des Fußteils gesperrt und eine erhöhte Sicherheit für den Prothesennutzer aufgrund der maximalen Kontrolle über das Fußteil ermöglicht.

Das Vorliegen oder Nichtvorliegen eines Bodenkontaktes kann über die Erfassung einer Bodenreaktionskraft ermittelt werden, wobei die Bodenreaktionskraft über einen Kraftsensor an dem Fußteil, dem Oberteil und/oder einer proximalen Prothesenkomponente ermittelt wird. Die Bodenreaktionskraft kann beispielsweise über einen Kontaktschalter, eine Druckmessfolie und/oder einen Dehnmessstreifen ermittelt werden. Die Anordnung eines Bodenreaktionskraftsensors zur Ermittlung des Zustandes, ob ein Bodenkontakt vorliegt oder nicht vorliegt, ist eine einfache und zuverlässige Möglichkeit, den jeweils vorliegenden Bewegungszustand zu detektieren. Grundsätzlich kann davon ausgegangen werden, dass ab Unterschreiten eines Schwellwertes einer Axialbelastung auf den Prothesenfuß keine Standphase mehr vorliegt oder zumindest die Standphase beendet werden soll, also dass das Körpergewicht auf die kontralaterale Seite verlagert wurde. Soweit noch ein ausreichend großer Axialkraftanteil vorhanden ist, kann von einem Bodenkontakt ausgegangen werden, sodass eine entsprechende Widerstandssteuerung gegen eine Plantarflexion und/oder Dorsalflexion vorgenommen werden kann. Ab Unterschreiten eines Schwellwertes kann davon ausgegangen werden, dass die Standphase eingeleitet worden ist bzw. gerade eingeleitet wird, sodass gegebenenfalls unterschiedliche Widerstände in die jeweilige Bewegungsrichtung eingestellt werden können. Die entsprechenden Kraftsensoren oder Momentensensoren können direkt an dem Fußteil, an dem Oberteil proximal der Schwenkachse oder aber an einer proximalen Prothesenkomponente, die mit dem Oberteil und damit auch mit dem Unterteil verbunden ist, angeordnet sein. Es können auch mehrere Sensoren zur Ermittlung des Bodenkontaktes über eine Bodenreaktionskraft oder ein entsprechendes Moment an dem Prothesenfuß oder der proximalen Prothesenkomponente angeordnet sein. Alternativ oder ergänzend kann das Vorliegen oder Nichtvorliegen eines Bodenkontaktes über zumindest einen Beschleunigungssensor an dem Fußteil, dem Oberteil und/oder der proximalen Prothesenkomponente ermittelt werden. Während in der Standphase in der Regel keine Bewegung des Prothesenfußes oder zumindest Teile des Prothesenfußes relativ zu dem Boden durchgeführt werden, wird in der Schwungphase der Prothesenfuß bewegt, insbesondere beschleunigt. Die Beschleunigung kann dabei sowohl in Vertikalrichtung als auch in Horizontalrichtung erfolgen, ebenso ist es möglich, die Vertikalbeschleunigung oder Horizontalbeschleunigung separat zu ermitteln. Beispielsweise beim Treppensteigen kann eine reine Vertikalbeschleunigung durch das senkrechte Anheben des Prothesenfußes auftreten. Nach dem Anheben oder nach einem nahezu vollständigen Entlasten kann durch eine Vorwärtsbewegung eine nahezu horizontale Beschleunigung des Prothesenfußes auftreten und über einen entsprechenden Beschleunigungssensor ermittelt werden.

Eine Weiterbildung der Erfindung sieht vor, dass eine Stellung des Fußteils relativ zu dem Oberteil und/oder eine Verschwenkbewegung des Fußteils zu dem Oberteil ermittelt wird und der Widerstand gegen eine Verschwenkung in Abhängigkeit von der ermittelten Stellung und/oder der ermittelten Verschwenkbewegung verändert wird. Der Knöchelgelenkswinkel oder der Winkel zwischen dem Oberteil und dem Unterteil kann beispielsweise über einen Knöchelsensor oder eine Ermittlung aus Raumlagedaten des Oberteils und des Unterteils ermittelt werden. Die Verschwenkbewegung des Fußteils relativ zu dem Oberteil kann durch die gleichen Sensoren ermittelt werden. Aus der jeweiligen Stellung ebenso wie der Veränderung der Stellung im Knöchelgelenk ist es möglich, Rückschlüsse auf das Vorliegen oder Nichtvorliegen eines Bodenkontaktes bzw. einer bestimmten Gangsituation zu ziehen, sodass diese Sensorwerte ebenfalls bei der Steuerung des Widerstandes der Verschwenkbewegung berücksichtigt werden.

Die Höhe der Bodenreaktionskraft kann gemessen und bei Erreichen oder Überschreiten eines Schwellwertes der Bodenreaktionskraft kann der Schwenkwiderstand erhöht oder eine Verschwenkung blockiert werden. Die Bodenreaktionskraft kann, wie oben ausgeführt, direkt über zumindest einen Kontaktschalter, eine Druckmessfolie und/oder zumindest einen DMS und/oder über die Verformung eines elastischen Elementes ermittelt werden. Die Verformung des elastischen Elementes kann über einen Drucksensor, einen kapazitiven Sensor, eine Längenmessung, eine Volumenmessung oder eine Widerstandseinrichtung erfasst werden, so dass daraus auf die Bodenreaktionskraft geschlossen werden kann. Weiterhin kann ergänzend oder alternativ über die Erfassung einer rotatorischen und/oder translatorischen Relativposition zweier flexibel miteinander verbundener Komponenten oder des Fußteils und einer anderen Komponente ermittelt werden, ob eine Bodenreaktionskraft vorhanden ist und ggf. wie groß diese Bodenreaktionskraft ist. Alternativ oder ergänzend kann über einen Knöchelmomentsensor aus dem Moment und dem angenommenen oder ermittelten Krafteinleitungspunkt die entsprechende Bodenreaktionskraft errechnet werden. Ebenfalls besteht die Möglichkeit, dass der Verschwenkwiderstand verringert wird, beispielsweise wenn durch Absinken der Bodenreaktionskraft unter einen Schwellwert erkannt wird, dass eine Schwungphase eingeleitet wird oder dass ein Übergang von einem Gehen auf der Ebene zu einem Gehen in einem ansteigenden oder aufsteigenden Untergrund erfolgt. Ebenso ist es möglich, dass erkannt wird, dass sich der Prothesennutzer in einer Bewegung befindet, die einen Übergang von dem Gehen auf einer Ebene oder einer Rampe zu einer Treppe andeutet.

Der Schwellwert der Bodenkraft kann beispielsweise zwischen 5% und 50% des Körpergewichtes des Patienten eingestellt werden. Unterschreitet beispielsweise der Schwellwert der Bodenreaktionskraft die Hälfte des Körpergewichtes des Patienten, kann daraus gefolgert werden, dass eine Standphase aufgehoben und eine Schwungphase eingeleitet werden soll. Befindet sich der Prothesennutzer längere Zeit in der Standphase, also steht der Nutzer über einen längeren Zeitraum, kann aus einer Verringerung unterhalb eines Schwellwertes oder auch aus der Geschwindigkeit der Verringerung geschlossen werden, ob eine Absicht besteht, einen Schritt in eine Richtung vorzunehmen, also ob eine Bewegungsabsicht besteht oder nicht. In Kombination mit Raumlageinformationen des Oberteils, des Fußteils oder auch einer proximalen Prothesenkomponente kann dann auf die Art und Weise der zu erwartenden Bewegung geschlossen werden und eine Verringerung beispielsweise des Dorsalflexionswiderstandes erfolgen. Wird beispielsweise ein Schwellwert wesentlich unter 50% des Körpergewichtes des Patienten detektiert oder eine Änderung der Drehrate bzw. Winkelbeschleunigung des Oberteils bzw. Unterschenkelteils ermittelt oder befindet sich der Knöchelwinkel in der Standposition anders als während des Gehens, wird der Flexionswiderstand verringert, sodass ein Losgehen möglich ist.

Der Winkel zwischen dem Fußteil und dem Oberteil oder der proximalen Prothesenkomponente kann gemessen werden, wobei der gemessene Winkel oder der Winkelverlauf auf Grundlage der gemessenen Winkel und die ermittelte Raumlage des Oberteils oder der proximalen Prothesenkomponente zur Veränderung des Widerstandes verwendet werden. Die Verstellung des Widerstandes erfolgt in der Standphase, wobei aus dem Knöchelwinkel und dem Inertialwinkel des Oberteils bzw. der proximalen Prothesenkomponente auf die jeweilige Situation geschlossen wird und in Abhängigkeit von den erfassten Werten die Verstellung des Widerstandes erfolgt. Es besteht auch die grundsätzliche Möglichkeit, nur den Knöchelwinkel zu verwenden, also den Relativwinkel zwischen dem Fußteil und dem Oberteil bzw. der proximalen Prothesenkomponente und daraus die entsprechend Verstellung des Widerstandes zu generieren.

Der maximale Dorsalflexionswinkel zwischen dem Oberteil und dem Fußteil kann in Abhängigkeit von der Untergrundneigung und/oder der Absatzhöhe verstellt werden. Die Verstellung eines solchen Dorsalflexionsanschlages kann über mehrere Teilschritte bis zum maximalen Dorsalflexionswinkel erfolgen. Je höher der Absatz ist, also je größer die Absatzhöhe und damit eine Veränderung der Nullpunktlage des Fußteils relativ zu dem Oberteil ist, desto mehr muss der Dorsalflexionsanschlag verstellt werden. Der gesamte mögliche Dorsalflexionswinkel muss sich dabei nicht verändern. Wird beispielsweise eine größere Absatzhöhe verwendet, wird der Dorsalflexionsanschlag in Plantarflexionsrichtung verstellt, um einen gleichen maximalen Dorsalflexionswinkel zu erzeugen. Wird die Absatzhöhe verringert, wird der Dorsalflexionsanschlag vorteilhafterweise in Dorsalflexionsrichtung verstellt, wobei die Verstellung über mehrere Schritte in einzelnen Teilschritten erfolgt, um eine langsame Anpassung an die geänderten äußeren Bedingungen zu ermöglichen und dadurch die Anpassung für den Prothesennutzer möglichst angenehm zu gestalten.

Bei Erreichen eines Grenzwertes für eine Neigung des Oberteils oder der proximalen Prothesenkomponente kann eine weitere Dorsalflexion unterbunden werden, dies gilt auch bei Erreichen eines Grenzwertes des Winkels zwischen dem Oberteil und dem Unterteil, wobei die Dorsalflexion jeweils bei Erreichen des einen oder anderen Kriteriums unterbunden wird. Das jeweils früher eintretende Kriterium ist somit ausschlaggebend, wodurch eine maximale Sicherheit für den Prothesennutzer erreicht wird.

Bei einem Stehen kann eine Dorsalflexion bei Erreichen eines festgelegten Schwellwertes der Raumorientierung der proximalen Prothesenkomponente gesperrt werden.

Beim Stehen kann nach Erreichen eines festgelegten Schwellwertes der Raumorientierung des Oberteils oder der proximalen Prothesenkomponente eine weitere Dorsalflexion gesperrt werden. Das Stehen kann beispielsweise durch Analyse eines Verlaufes des Krafteinleitungspunktes erfolgen, also das Wandern eines Krafteinleitungspunktes zwischen einem Vorderfußbereich und einem Fersenbereich. Befindet sich der Krafteinleitungspunkt innerhalb eines Bereiches zwischen zwei Auflagepunkten und besteht eine Axiallast auf beiden Auflagepunkten, kann eine weitere Dorsalflexion gesperrt werden, um eine maximale Stabilität zu erreichen. Ebenso kann bei einer geringen Geschwindigkeit des Krafteinleitungspunktes davon ausgegangen werden, dass sich der Prothesennutzer nicht in einer Gehsituation befindet, sondern in einer Stehsituation, sodass nur eine gewisse Dorsalflexion während der Stehfunktion und Stehsituation zugelassen wird.

Die Sperrung der Dorsalflexion kann bei einer Vergrößerung einer Bodenreaktionskraft auf oberhalb von 50% des Patientengewichtes, und/oder bei einer Änderung der Drehrate des Oberteils und/oder beim Auftreten einer Winkelbeschleunigung des Oberteils aufgehoben werden. Bei Eintreten zumindest eines dieser Kriterien kann darauf geschlossen werden, dass die Stehsituation aufgegeben werden soll und dass ein Schritt nach vorn oder hinten veranlasst wird.

Beim Gehen auf einem abfallenden Untergrund wird der Dorsalflexionsanschlag vorteilhafterweise in Richtung auf einen vergrößerten Dorsalflexionswinkel verstellt, der Anschlag wird also in Dorsalflexionsrichtung verstellt, um ein möglichst natürliches Gangbild zu erreichen. Informationen über die Untergrundneigung können beispielsweise aus der Raumlageinformation des Fußteils bei einem vollflächigen Kontakt der Schuhsohle oder Fußsohle mit dem Untergrund ermittelt werden. Sind beispielsweise zwei Kraftsensoren im Fußsohlenbereich angeordnet, kann darüber ermittelt werden, in welcher Phase des Stehens oder der Standphase sich ein Fußteil befindet. Ebenfalls kann über die Ermittlung eines Knöchelgelenkverlaufes und eines Knöchelmomentverlaufes, gegebenenfalls in Verbindung mit IMU-Daten des Oberteils und/oder des Unterteils, Rückschlüsse auf den jeweiligen Untergrund und die jeweilige Untergrundneigung erlangt werden. Befindet sich ein Nutzer der Protheseneinrichtung auf einer nach oben führenden Rampe, wird der Dorsalflexionsanschlag verstellt und eine vergrößerte Dorsalflexion ermöglicht.

Beim Gehen auf abfallendem Untergrund kann der Dorsalflexionsanschlag gegenüber dem Gehen in der Ebene um bis zu 500% vergrößert werden, befindet sich der Dorsalflexionsanschlag normalerweise ausgehend von einem Ausgangswert bei beispielsweise 10°, kann dieser beim Gehen auf einer Rampe verdoppelt oder verfünffacht werden, also ein Dorsalflexionswert von 20° bis 50° eingestellt werden.

In einer Weiterbildung der Erfindung ist es vorgesehen, dass das Fußteil in einer Schwungphase in seine Ausgangsstellung zurückbewegt wird, dies kann durch passive Energiespeicher, wie Federn geschehen, die in der Standphase bei der Abrollbewegung aufgeladen, gegebenenfalls in der anfänglichen Schwungphase gesperrt und nach dem Durchschwingen freigegeben werden, sodass der Prothesenfuß beim Aufsetzen, also beim inertialen Bodenkontakt wieder entsprechend bewegt wird. Alternativ oder ergänzend zu einer Rückstellung über einen passiven Energiespeicher kann diese über einen aktiven Antrieb in Gestalt eines Motors erfolgen.

Eine Weiterbildung der Erfindung sieht vor, dass bei einer senkrechten Orientierung des Oberteils der Widerstand der Widerstandseinrichtung auf einen Minimalwert eingestellt wird, bevorzugt wird der Widerstand auf Null gesetzt, so dass kein zusätzlicher Widerstand durch die Widerstandseinrichtung gegen eine Verschwenkung in Plantarflexionsrichtung und in Dorsalflexionseinrichtung bereitgestellt wird. Eine solche Steuerung ist sowohl beim Gehen in der Ebene als auch beim Gehen auf Rampen, sowohl aufwärts als auch abwärts vorteilhaft, so dass bei einem sogenannten Nulldurchgang des Unterschenkels oder des Oberteils eine freie oder nahezu freie Verschwenkung des Fußteils ermöglicht. Bei einem Übergang von einer Plantarflexion zu einer Dorsalflexion mit einem eingestellten und/oder modulierten Widerstand erfolgt der Momentendurchgang bei dem Nulldurchgang der Oberteils oder einer senkrechten Orientierung des Oberteils oder des Unterschenkels.

Nachfolgend werden Ausführungsbeispiele der Erfindung anhand der Figuren näher erläutert. Es zeigen:
- Figur 1 -: eine Seitenansicht eines Prothesenfußes mit proximalen Komponenten;
- Figuren 2 bis 4 -: schematische Darstellungen von Prothesen mit Prothesenfüßen;
- Figur 5 -: ein Steuerungsbeispiel;
- Figur 6 -: eine Knöchelwinkelverkauf beim Gehen auf einer Schräge;
- Figur 7 -: eine schematische Darstellung einer Gangsituation; sowie
- Figuren 8 und 9 -: Widerstandsverläufe über dem Unterschenkelwinkel .

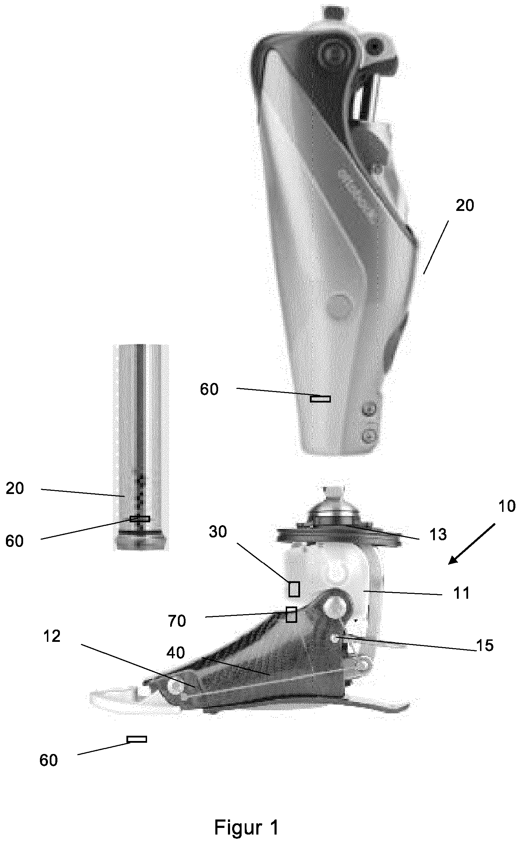

**In** der Figur 1 ist in einer Seitenansicht ein Prothesenfuß 10 mit einem Oberteil 11 und einem Fußteil 12 dargestellt, die um eine Schwenkachse 15 schwenkbar miteinander gekoppelt sind. An dem proximalen Ende des Oberteils 11 ist ein Befestigungselement 13 in Gestalt eines Pyramidenadapters angeordnet. Über das Befestigungselement 13 ist es möglich, proximale Prothesenkomponenten 20, beispielsweise ein Unterschenkelrohr oder ein Unterschenkelteil mit einem Prothesenkniegelenk und einem integrierten Dämpfer an dem Prothesenfuß 10 lösbar zu befestigen. In dem dargestellten Ausführungsbeispiel weist der Prothesenfuß 10 genau eine Schwenkachse 15 zur Ausbildung eines Knöchelgelenks auf. An der Unterseite des Fußteils 12 ist ein Sensor 60 angeordnet, der einen Bodenkontakt des Fußteils 12 detektiert. Der Sensor 60 kann als elektrisches Kontaktelement, als Drucksensor, als Dehnmessstreifen oder eine andere Ausgestaltung eines Kraftsensors ausgebildet sein. In dem dargestellten Ausführungsbeispiel ist der Bodenkontaktsensor 60 im Vorderfußbereich des Fußteils 12 angeordnet. Alternativ oder ergänzend kann ein Bodenkontaktsensor auch im Fersenbereich oder Mittelfußbereich angeordnet sein. Ein Bodenkontakt kann auch über andere Sensoren 60 ermittelt werden, beispielsweise über einen Axialkraftsensor, der an dem Oberteil 11 oder an dem Befestigungselement 13 angeordnet ist. Ebenso kann eine entsprechende Sensoreinrichtung 60 an der proximalen Prothesenkomponente 20 angeordnet sein, beispielsweise als Dehnmessstreifen oder Drucksensor an einer Verbindungsstelle an dem Unterschenkelrohr,dem Unterschenkelteil und/oder einem Knieteil.

Innerhalb des Prothesenfußes 10 ist darüber hinaus eine Widerstandseinrichtung 40 angeordnet, mit der es möglich ist, eine Plantarflexion oder eine Dorsalflexion mit einem einstellbaren Widerstand zu beaufschlagen. Die Widerstandseinrichtung 40 kann als Hydraulikdämpfer, als Dämpfer auf Basis einer magnetorheologischen Flüssigkeit, als mechanische Bremse oder als entsprechend im Generatorbetrieb betriebener Motor ausgebildet sein. Um auf der Grundlage von Sensordaten und Daten einer inertialen Messeinheit 30 (IMU) die Widerstandseinrichtung 40 einzustellen, sind die Sensoren bzw. die Sensorik 30, 60 mit einer Steuerungseinrichtung 70 gekoppelt, beispielsweise über eine Kabelverbindung oder drahtlos über eine Funkverbindung, um in Abhängigkeit von den Sensordaten den Widerstand gegen eine Verschwenkung in die eine oder andere Richtung um die Schwenkachse 15 zu verstellen oder einzustellen.

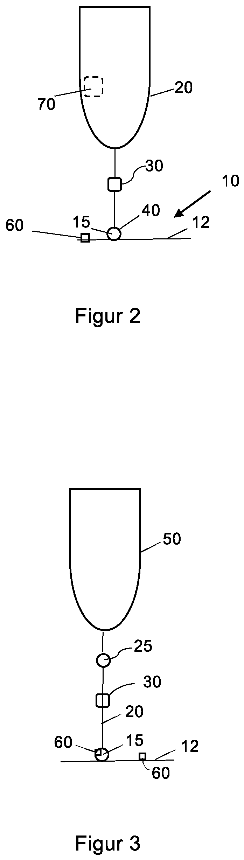

In der Figur 2 ist in einer schematischen Darstellung die Protheseneinrichtung mit dem Prothesenfuß 10, der proximalen Prothesenkomponente 20 in Gestalt eines Unterschenkelschaftes sowie dem an dem Unterschenkelrohr befestigten Inertialwinkelsensor 30 oder IMU gezeigt. Der Prothesenfuß 10 ist über ein Gelenk um die Achse 15 verschwenkbar an dem Unterschenkelschaft 20 gelagert. Das Oberteil 11 und das Befestigungselement 13 sind aus Gründen der Übersichtlichkeit nicht eingezeichnet. An dem Unterschenkelrohr ist die IMU 30 entweder dauerhaft oder abnehmbar festgelegt ist. An dem Unterschenkelschaft 20 ist darüber hinaus eine Steuerungseinrichtung 70 angeordnet, die mit dem Bodenkontaktsensor 60, der IMU 30 sowie der Widerstandseinrichtung 40 gekoppelt ist. Die Anordnung des Inertialwinkelsensor 30 oder der IMU kann entweder integriert in oder lösbar befestigt an einem Teil des Prothesenfußes 10 erfolgen, der mit dem Unterschenkelrohr und dem Unterschenkelschaft 20 verbunden ist. Ebenfalls ist es möglich, den Sensor 60 nicht an der Ferse, sondern an einem Mittelfußbereich, dem Oberteil oder an dem Gelenk anzuordnen. Alternativ sind mehrere Sensoren 60 an dem Fußteil 12 angeordnet.

In der Figur 3 ist eine weitere Protheseneinrichtung schematisch dargestellt, bei der eine zweite proximale Prothesenkomponente 50 an dem Unterschenkelteil 20 angeordnet ist. Die zweite proximale Prothesenkomponente 50 ist beispielsweise ein Oberschenkelschaft mit einem Verbindungsrohr zu einem Prothesenkniegelenk 25. In dem dargestellten Ausführungsbeispiel ist die IMU 30 wieder an dem Unterschenkelrohr 20 angeordnet, alternativ kann der Inertialwinkelsensor 30 und gegebenenfalls auch die Steuerungseinrichtung 70 integriert oder lösbar befestigt an dem Kniegelenk 25 oder dem Oberschenkelschaft 50 oder einem Verbindungsteil zwischen dem Oberschenkelschaft 50 und dem Kniegelenk 25 angeordnet sein. Ebenfalls kann die IMU 30 an einem Oberteil eines Prothesenkniegelenkes angeordnet sein. In Verbindung mit einem Winkelsensor, der den Winkel zwischen dem Unterschenkelteil 20 und der proximalen Komponente 50 erfasst, kann aus der Raumlage der zweiten proximalen Komponente 50 die Raumlage des Unterschenkelteils 20 detektiert werden. In dem Ausführungsbeispiel der Figur 3 sind darüber hinaus Belastungs-, Beschleunigungs- und/oder Winkelsensoren 60 vorgesehen, die an dem Fußteil 12 und dem Knöchelgelenk in dem Bereich der Schwenkachse 15 angeordnet sind. Die Belastungssensoren 60 sind beispielsweise Axialkraftsensoren, Drucksensoren, z.B. in Gestalt von Kontaktschaltern, Druckmessfolien und/oder einem DMS und/oder Momentensensoren, um die jeweilige Belastung auf die Protheseneinrichtung zu erfassen. Die Sensoren 30, 60 sind mit der nicht dargestellten Steuerungseinrichtung 70 gekoppelt.

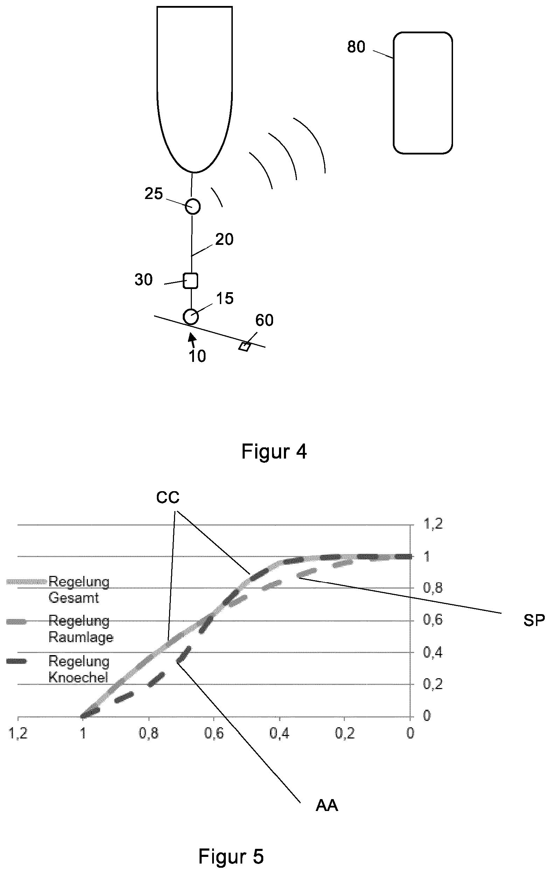

In der Figur 4 ist die Steuerungseinrichtung 70 von dem Inertialwinkelsensor 30 getrennt und räumlich separiert ausgebildet und über eine drahtlose Verbindung mit dem Prothesenfuß 10 gekoppelt. Die Datenübertragung von der Sensorik zu der Steuerungseinrichtung 70 kann über Funk, WLAN, Bluetooth^{®}, NFC oder andere Übertragungswege erfolgen.

Mit den Sensoren 60 sowie der IMU 30 ist es möglich, den Widerstand gegen eine Verschwenkung präzise zu steuern. Der eine Sensor 30 ermittelt den Bodenkontakt des Fußteils 12 und stellt somit sicher, dass sich der Prothesenfuß 10 in der Standphase befindet. Gleichzeitig wird die Raumlage des Fußteils 12 und/oder des Oberteils 11 über die IMU 30 ermittelt. Wird der Bodenkontakt des Fußteils 12 detektiert, beispielsweise durch einen an der Sohle befestigten Drucksensor 60, wird die gleichzeitig ermittelte Raumlage des Fußteils 12 und/oder des Oberteils 11 erfasst und zur Steuerung des Widerstandes herangezogen. Dadurch ergibt sich eine überlagerte Steuerung auf der Grundlage der Raumlage des Fußteils 12 und/oder des Oberteils 11 in Verbindung mit der Detektion der Standphase, sodass sich eine erhöhte Zuverlässigkeit bei der Einstellung des jeweiligen Widerstandes erreichen lässt. Diese erhöhte Zuverlässigkeit tritt insbesondere bei Situationsübergängen ein, also bei Veränderungen im Untergrund, beispielsweise bei Änderung der Untergrundneigung oder bei Veränderung des Schrittrhythmus, beispielsweise wenn vom Gehen in der Ebene auf das Treppensteigen oder von dem Rampengehen in das Gehen in der Ebene oder das Treppensteigen gewechselt wird. Dabei kann insbesondere auch die Stellung des Oberteils 11 relativ zu dem Fußteil 12 als weitere Einflussgröße und Sensorwert mit in Betracht gezogen werden. Dazu ist ein Knöchelgelenkswinkel zu ermitteln, beispielsweise über einen Winkelsensor 60 direkt an der Schwenkachse 15 oder über die IMU 30 an dem Fußteil 12 und dem Oberteil 11 bzw. einer daran befestigten proximalen Prothesenkomponente 20.

Wird beispielsweise bei Nichtvorliegen eines Bodenkontaktes des Fußteils 12, was über die Bodenreaktionskraft über einen Kraftsensor an dem Fußteil 12, dem Oberteil 11 und/oder der proximalen Prothesenkomponente 20 ermittelt wird, eine Raumlageänderung über eine IMU 30 detektiert, sei es eine Änderung der Raumlage der proximalen Prothesenkomponente 20 oder des Oberteils 11 oder des Fußteils 12, ergibt sich daraus für die Steuerung, dass in einer Schwungphase, in der sich der Prothesenfuß offenbar befindet, ein anderer Widerstand in der Widerstandseinrichtung 40 eingestellt wird als in der Standphase. Während bei einer Raumlageänderung beispielsweise des Fußteils 12 bei Vorliegen einer Standphase darauf geschlossen werden kann, dass eine Abrollbewegung des Prothesenfußes stattgefunden und eine terminale Standphase angenommen wird, bei der die Dorsalflexion unterbunden werden soll und somit ein erhöhter Dorsalflexionswiderstand bereitgestellt werden muss, kann bei der gleichen Verlagerung des Fußteils 11 im Raum während der Schwungphase eine Dorsalflexion erwünscht sein, um ein freies Durchschwingen des Prothesenfußes zu ermöglichen, sodass dabei der Dorsalflexionswiderstand verringert wird und gegebenenfalls ein Aktuator aktiviert wird, um eine Dorsalflexion des Fußteils 12 zu ermöglichen. Der Aktuator kann beispielsweise als ein passiver Energiespeicher ausgebildet sein, der in der Schwungphase freigeschaltet wird, sodass eine Rückstellung zumindest in eine neutrale Lage erfolgt. Alternativ ist der Aktuator ein aktiver Antrieb oder Motor, der das Fußteil in seine Ausgangsposition zurückbewegt.

Das Nichtvorliegen eines Bodenkontaktes kann alternativ oder ergänzend über einen Beschleunigungssensor 60 an dem Fußteil 12, dem Oberteil 11 und/oder der proximalen Prothesenkomponente 20 ermittelt werden. Findet beispielsweise trotz einer Entlastung des Fußteils 12 oder des Oberteils 11 keine Beschleunigung einer der beiden Komponenten statt, kann auf einen statischen Zustand geschlossen werden, beispielsweise das Vorliegen einer Standphase. Bei bei einer reinen Vertikalbeschleunigung ohne Horizontalanteil kann auf ein Anheben des Prothesenfußes ohne Vorwärtsbewegung geschlossen werden, beispielsweise zum Überschreiten eines Hindernisses oder zum Treppensteigen. Auf der Grundlage der Beschleunigungsdaten des Beschleunigungssensors 60 kann entweder allein oder in Ergänzung mit einem Kraftsensor darauf geschlossen werden, ob ein Bodenkontakt vorhanden ist und in welcher Phase der Bewegung, sofern eine Bewegung erkannt wird, sich der jeweilige Prothesenfuß befindet.

Neben den Absolutlagen und den Beschleunigungen ist es ebenfalls vorgesehen, dass die Stellung des Fußteils 12 relativ zu dem Oberteil 11 und/oder eine Verschwenkbewegung des Fußteils 12 relativ zu dem Oberteil 11 ermittelt wird. Die Stellung kann über einen Winkelsensor 60 ermittelt werden, die Verschwenkbewegung wird über die zeitliche Ableitung der Winkelsensordaten oder aber über einen Beschleunigungssensor detektiert. Auch die Verstellbewegung sowie die Stellung von dem Fußteil 12 relativ zu dem Oberteil 11 und damit auch zu der proximalen Prothesenkomponente 20, die in Regel starr an dem Oberteil 11 gekoppelt ist, trägt zur Zuverlässigkeit der Steuerung bei.

In der Figur 5 ist ein Beispiel einer Steuerung basierend auf der Raumlage des Prothesenfußes bzw. dessen Komponenten und des Winkels zwischen dem Fußteil 12 und dem Oberteil 11 aufgezeigt. Eine reine Regelung über den Knöchelwinkel, also die Stellung des Fußteils 12 relativ zu dem Oberteil 11, ist in der unteren Kurve mit der unterbrochenen Linie AA dargestellt. Die Regelung allein auf Raumlage ist in der unterbrochenen Linie SP dargestellt, die Gesamtregelung ist die durchgezogene Linie CC im oberen Verlauf. Es wurden jeweils, je nach erkannter Situation, ein Ziel-Knöchelwinkel und eine Zielorientierung des Oberteils 11 bzw. des Unterschenkelteils 20 vorgegeben. Bei vollständiger Übereinstimmung des jeweiligen Ist-Winkels mit dem Ziel-Winkel ergibt sich der Wert 1, sodass keine Verstellung notwendig ist, nach Abweichungsgrad verändern sich der Wert und damit auch die notwendige Verstellung der Widerstandseinrichtung. Würde beispielsweise die Steuerung nur auf Grundlage der Raumlagekurve SP erfolgen, wäre bei einem Wert 0,4 eine vergleichsweise geringe Verstellung auf ungefähr 0,8 des Widerstandswertes notwendig, wohingegen bei einer alleinigen Steuerung auf Grundlage des Knöchelwinkels und der Kurve AA eine Verstellung auf 0,95 notwendig wäre. Umgekehrt wäre bei einem Wert von 0,8 bei alleiniger Steuerung über den Knöchelgelenkswinkel und die Kurve A-A eine Verstellung auf 0,2 notwendig, während bei der Regelung über die Raumlage aufgrund der Kurve SP eine Verstellung auf 0,35 erfolgen würde. Für die jeweilige Steuerung der Widerstandseinrichtung 40 wird jeweils der sicherere Ausgabewert eingestellt, sodass sich die obere Kurve CC ergibt, die in der durchgezogenen Linie dargestellt ist. Insbesondere bei Übergängen von geneigten Untergründen zum Treppensteigen oder bei anderen Übergangssituationen, beispielsweise vom Gehen in der Ebene zum Rampe-Gehen ist eine solche Kombination der Steuerung der Widerstandseinrichtung 40 aufgrund von Raumlage- und Relativwinkelwerten besonders vorteilhaft.

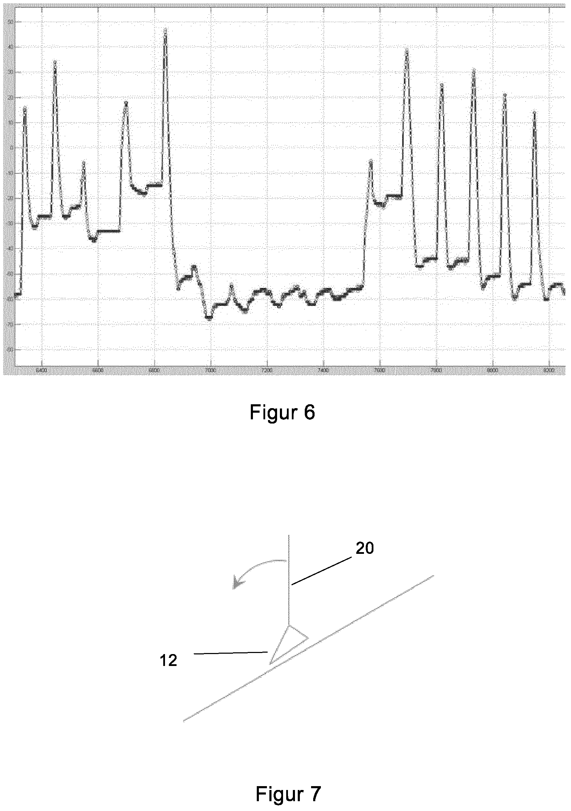

In der Figur 6 ist der Knöchelgelenkswinkel, also der Winkel zwischen dem Fußteil 12 und dem Oberteil 11 über den Verlauf mehrerer Schritte dargestellt. Ausgehend von einer Ausgangsposition, die mit "0" gekennzeichnet ist, wird beim normalen Gehen in der Ebene eine mehr oder weniger gleichmäßige Amplitude oberhalb und unterhalb einer Ausgangsstellung ausgeführt. Positive Werte zeigen eine Plantarflexion an, negative Werte eine Dorsalflexion. Der Übergang von dem Gehen in der Ebene zum Rampe-Aufwärts-Gehen findet bei dem Wert von ungefähr 6900 statt. Das Oberteil muss beim Gehen auf einer Rampe bergauf weiter in Richtung auf das Fußteil verlagert werden, sodass eine erhöhte Dorsalflexion stattfindet. Diese wird für das Gehen auf der Rampe nahezu unverändert gehalten, bis bei 7500 erneut eine ebene Stelle erreicht wird und die Widerstandseinrichtung wieder an das Gehen in der Ebene angepasst werden kann.

Die Verstellung der Widerstandseinrichtung 40 kann auch dazu genutzt werden, um den Ausgangswert für eine Verstellung um eine Ausgangslage zu verstellen. Durch die Verschiebung des Nullpunktes, um den die Steuerung des Widerstandes der Protheseneinrichtung herum ausgeführt wird, kann beispielsweise eine Anpassung an unterschiedliche Neigungswinkel im Untergrund oder auch an unterschiedliche Absatzhöhen vorgenommen werden. Sowohl für die Plantarflexion als auch für die Dorsalflexion können Anschläge eingestellt werden, bei denen die Widerstandseinrichtung 40 einen maximalen Wert aufweist und einnimmt, um eine weitere Flexion zu unterbinden. So kann bei Erreichen eines Grenzwertes für eine Neigung des Oberteils 11 oder der sich daran anschließenden proximalen Prothesenkomponente 20 eine weitere Dorsalflexion unterbunden werden. Der Grenzwert für die Neigung wird dabei über die IMU 30 ermittelt oder aus einer Kombination der Raumlagewerte des Fußteils und eines Knöchelwinkels. Eine entsprechende Sperrung der Dorsalflexion kann auch bei Erreichen eines maximalen Dorsalflexionswinkels erreicht werden, wobei das jeweils früher eintretende Kriterium, also die maximale Raumlage oder der maximale Dorsalflexionswinkel ausschlaggebend dafür ist, wann eine Sperrung erreicht wird.

Figur 7 zeigt eine schematische Darstellung des Prothesenfußes mit dem Fußteil 12, das an der proximalen Prothesenkomponente 20 gelenkig gelagert ist. Das Fußteil 12 steht auf einer nach unten geneigten Ebene. Befindet sich der Nutzer der Protheseneinrichtung nicht in Bewegung, sondern steht, kann eine Dorsalflexion beispielsweise bei festgestellter senkrechter Positionierung trotz Nichterreichen der üblichen Ausgangsstellung des Fußteils 12 relativ zu dem Oberteil 11 eine Sperrung der Dorsalflexion erfolgen. Wird eine Veränderung der Absatzhöhe oder eine sich verändernde Untergrundneigung festgestellt, beispielsweise aufgrund signifikanter Abweichungen der Bewegungsmuster und der Winkelverläufe, wie anhand der Figur 6 erläutert, kann die Anpassung und Verlagerung der Nullstellung, um die herum eine Dorsalflexion und Plantarflexion erfolgt, schrittweise durchgeführt werden, sodass eine Anpassung an eine geänderte Untergrundneigung sukzessive erfolgt und die Änderungsamplituden nicht so groß ausfallen. Wird das Gehen auf einem geneigten Untergrund erkannt, kann die Dorsalflexion entsprechend verstellt werden. Beim Gehen auf einem absteigenden Untergrund, wie in der Figur 7 dargestellt, kann der Dorsalflexionsanschlag in Richtung auf einen verringerten Dorsalflexionswinkel verstellt werden, bei einer umgekehrten Bewegung, also aus bergauf, kann der Dorsalflexionsanschlag in Richtung auf einen vergrößert Dorsalflexionswinkel verstellt werden, um den Bewegungsablauf gemäß Figur 6 Rechnung zu tragen.

Jeweils in der Schwungphase kann das Fußteil 12 in eine Ausgangsstellung zurückbewegt werden, die die sogenannte Nulllage bildet, um die herum eine Dorsalflexion und Plantarflexion mit dem entsprechenden Widerstandsprofil durchgeführt wird.

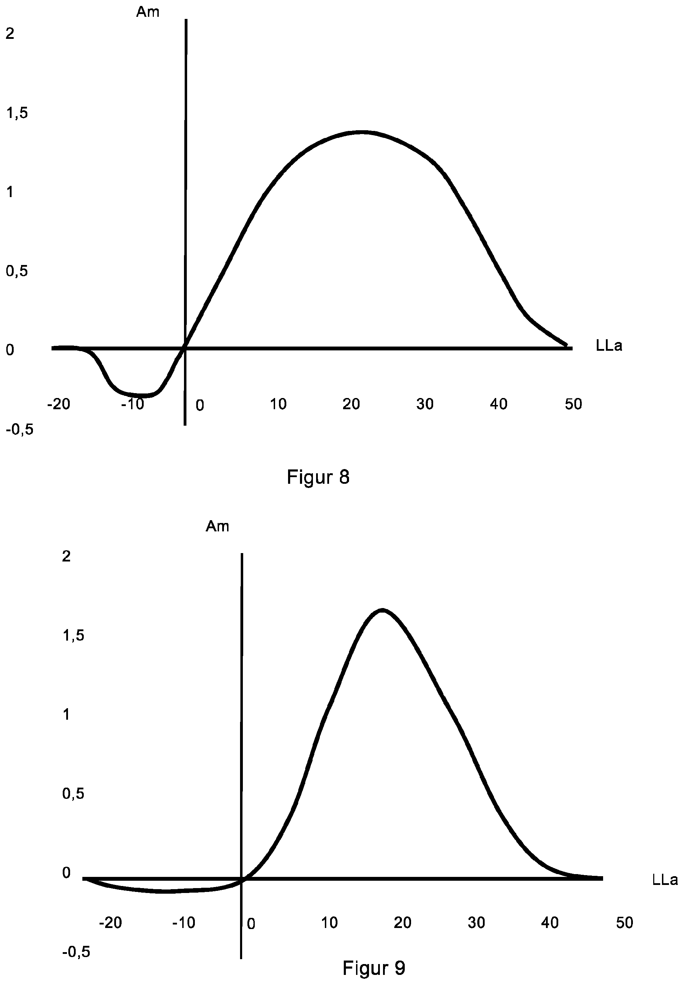

In der Figur 8 ist eine schematische Darstellung eines Widerstandsverlaufes oder eines Knöchelmomentes Am über den Unterschenkelwinkel LLa dargestellt. Der Unterschenkelwinkel LLa entspricht im Wesentlichen der Orientierung des Oberteils 11 des Prothesenfußes, da eine proximale Komponente wie ein Unterschenkelrohr oder das Unterteil eines Prothesenkniegelenkes eine geradlinige Verlängerung des Oberteils darstellt. Bei einem Unterschenkelwinkel von 0° befinden sich das Oberteil und das Unterschenkelteil in einer Ausgangsstellung oder senkrechten Stellung, negative Werte entsprechen einer Plantarflexion des Fußteils, positive Werte einer Dorsalflexion.

Über den Unterschenkelwinkel LLa ist der Widerstand oder das Knöchelmoment Am aufgetragen, das einen Widerstand gegen eine Verlagerung des Fußteils relativ zu dem Oberteil oder dem Unterschenkel bereitstellt. In der Figur 8 ist das Bergabgehen auf einer Rampe dargestellt. Nach dem heel strike und einer vergleichsweise großen Plantarflexion bewegt sich das Fußteil in Richtung einer Ausgangsposition oder einer Nullstellung, wobei das Knöchelmoment zunächst zunimmt und bei einer Annäherung an die Ausgangsstellung wieder abnimmt, bis es bei Erreichen der Ausgangsstellung und einer senkrechten Orientierung des Oberteils einen Minimalwert erreicht. Ein Minimalwert kann auch mehrfach während eines Schrittzyklusses erreicht werden. Bei einer zunehmenden Dorsalflexion wird das Knöchelmoment und der Widerstand Am vergrößert, bis ein maximaler Widerstandswert bei einem Unterschenkelwinkel von circa 20 bis 30° zur Senkrechten erreicht wird. Anschließend sinkt das Knöchelmoment bei zunehmender Dorsalflexion.

In der Figur 9 ist der Widerstandsverlauf Am über den Unterschenkelwinkel beim Rampe Aufwärts Gehen dargestellt. Beim Rampe Aufwärts Gehen ist in der Regel keine Plantarflexion zu erwarten, sodass ein Aufsetzen des Fußes auf einen aufsteigenden Untergrund ausgehend von einer Normalstellung in einer Dorsalflexion mündet. Ausgehend davon erfolgt eine Vergrößerung des Widerstandes Am oder des Knöchelmomentes im Vergleich zur Rampe Abwärts Gehen wesentlich schneller, sodass der Maximalwert in einem Bereich zwischen 10° und 20° Dorsalflexionen des Fußteils erfolgt. Bei einer zunehmenden Dorsalflexion wird der Widerstand Am verringert, bis ein maximaler Endanschlag erreicht ist.

## Patentansprüche

1. Verfahren zur Steuerung eines Prothesenfußes (10), der ein Oberteil (11) mit einem Befestigungselement (13) für eine proximale Prothesenkomponente (20) und ein um eine Schwenkachse (15) schwenkbar daran gelagertes Fußteil (12) aufweist, mit einer Widerstandseinrichtung (40), mit der einer Verschwenkbewegung des Fußteils (12) relativ zu dem Oberteil (11) ein einstellbarer Widerstand (Am) entgegengesetzt wird und mit einer Steuerungseinrichtung (70), die mit der Widerstandseinrichtung (40) und mit zumindest einem Sensor (60) gekoppelt ist und über die in Abhängigkeit von Sensordaten der Widerstand gegen eine Verschwenkung eingestellt wird, wobei über zumindest einen Sensor (60) ein Bodenkontakt des Fußteils (12) mit dem Boden ermittelt wird, wobei die Raumlage des Fußteils (12) und/oder des Oberteils (11) über eine inertiale Messeinheit (30) ermittelt wird und wobei der Widerstand (Am) gegen eine Verschwenkung in Abhängigkeit von dem Vorliegen oder Nichtvorliegen eines Bodenkontaktes und der ermittelten Raumlage und/oder dem ermittelten Verlauf der Raumlage verändert wird, **dadurch gekennzeichnet, dass** eine Dorsalbewegung in Abhängigkeit von dem gemessenen Winkel zwischen dem Oberteil (11) und dem Fußteil (12) und/oder einem Raumlagewinkel ermöglicht oder unterbunden wird.

2. Verfahren nach Anspruch 1, **dadurch gekennzeichnet, dass** das Vorliegen oder Nichtvorliegen eines Bodenkontaktes über eine Bodenreaktionskraft ermittelt wird, wobei die Bodenreaktionskraft über zumindest einen Kraftsensor (60) an dem Fußteil (12), dem Oberteil (11) und/oder der proximalen Prothesenkomponente (20) ermittelt wird.

3. Verfahren nach Anspruch 1 oder 2, **dadurch gekennzeichnet, dass** das Vorliegen oder Nichtvorliegen eines Bodenkontaktes über zumindest einen Beschleunigungssensor (60) an dem Fußteil (12), dem Oberteil (11) und/oder der proximalem Prothesenkomponente (20) ermittelt wird.

4. Verfahren nach einem der voranstehenden Ansprüche, **dadurch**
**gekennzeichnet, dass** eine Stellung des Fußteils (12) relativ zu dem Oberteil (11) und/oder eine Verschwenkbewegung des Fußteils (12) zu dem Oberteil (11) ermittelt und der Widerstand gegen eine Verschwenkung in Abhängigkeit von der ermittelten Stellung und/oder Verschwenkbewegung verändert wird.

5. Verfahren nach einem der voranstehenden Ansprüche, **dadurch**
**gekennzeichnet, dass** die Bodenreaktionskraft über zumindest einen Kontaktschalter, über eine Verformung eines elastischen Elementes, über die Erfassung einer rotatorischen und/oder translatorischen Relativposition zweier flexibel miteinander verbundener Komponenten, über eine Druckmessfolie und/oder über einen Dehnungsmessstreife (DMS) als Sensor (60) ermittelt wird.

6. Verfahren nach einem der voranstehenden Ansprüche, **dadurch**
**gekennzeichnet, dass** die Höhe der Bodenreaktionskraft gemessen und bei Erreichen oder Überschreiten eines Schwellwertes der Bodenreaktionskraft der Verschwenkwiderstand verringert, erhöht oder eine Verschwenkung blockiert wird.

7. Verfahren nach Anspruch 6, **dadurch gekennzeichnet, dass** der Schwellwert zwischen 5% und 50% des Körpergewichtes des Patienten eingestellt wird.

8. Verfahren nach einem der voranstehenden Ansprüche, **dadurch**
**gekennzeichnet, dass** der Winkel zwischen dem Fußteil (12) und dem Oberteil (11) oder der proximalen Prothesenkomponente (20) gemessen und der gemessene Winkel oder Winkelverlauf und die ermittelte Raumlage des Oberteils (11) oder der proximalen Prothesenkomponente (20) zur Veränderung des Widerstandes verwendet werden.

9. Verfahren nach Anspruch 1, **dadurch gekennzeichnet, dass** der maximale Dorsalflexionswinkel zwischen dem Oberteil (11) und dem Fußteil (12) in Abhängigkeit von der Untergrundneigung und/oder Absatzhöhe verstellt wird.

10. Verfahren nach Anspruch 9, **dadurch gekennzeichnet, dass** die Verstellung eines Dorsalflexionsanschlages über mehrere Teilschritte bis zu dem maximalen Dorsalflexionswinkel erfolgt.

11. Verfahren nach einem der Ansprüche 8 bis 10, **dadurch gekennzeichnet, dass** bei Erreichen eines Grenzwertes für eine Neigung des Oberteils (11) oder der proximalen Prothesenkomponente (20) eine weitere Dorsalflexion und ab Erreichen eines Grenzwertes des Winkels zwischen dem Oberteil (11) und dem Fußteil (12) eine weitere Dorsalflexion unterbunden wird, wobei das früher eintretende Kriterium ausschlaggebend ist.

12. Verfahren nach einem der voranstehenden Ansprüche, **dadurch**
**gekennzeichnet, dass** beim Stehen eine Dorsalflexion bei Erreichen eines festgelegten Schwellwertes der Raumorientierung des Oberteils (11) oder der proximalen Prothesenkomponente (20) gesperrt wird.

13. Verfahren nach Anspruch 12, **dadurch gekennzeichnet, dass** die Sperrung der Dorsalflexion bei einer Erhöhung einer Bodenreaktionskraft auf über 50% des Patientengewichtes, bei einer Änderung einer Drehrate des Oberteils (11) und/oder Auftreten einer Winkelbeschleunigung des Oberteils (11) aufgehoben wird.

14. Verfahren nach einem der voranstehenden Ansprüche, **dadurch**
**gekennzeichnet, dass** beim Gehen auf abfallendem Untergrund ein Dorsalflexionsanschlag in Richtung auf einen vergrößerten Dorsalflexionswinkel verstellt wird.

15. Verfahren nach Anspruch 14, **dadurch gekennzeichnet, dass** beim Gehen auf abfallendem Untergrund der Dorsalflexionsanschlag gegenüber dem Gehen in der Ebene um 100% bis 500% vergrößert wird.

16. Verfahren nach einem der voranstehenden Ansprüche, **dadurch**
**gekennzeichnet, dass** das Fußteil (12) in einer Schwungphase in eine Ausgangstellung zurückbewegt wird.

17. Verfahren nach einem der voranstehenden Ansprüche, **dadurch**
**gekennzeichnet, dass** bei einer senkrechten Orientierung des Oberteils (11) der Widerstand (Am) der Widerstandseinrichtung (40) auf einen Minimalwert eingestellt wird.

## Claims

1. A method for controlling a prosthetic foot (10) which has an upper part (11) having a fastening element (13) for a proximal prosthesis component (20) and a foot part (12) mounted thereon so as to be pivotable about a pivot axis (15), having a resistance device (40) with which an adjustable resistance (Am) is presented against a pivoting movement of the foot part (12) relative to the upper part (11), and having a control device (70) which is coupled with the resistance device (40) and with at least one sensor (60) and by means of which the resistance against pivoting is set as a function of sensor data, wherein ground contact of the foot part (12) with the ground is determined by means of at least one sensor (60), wherein the spatial attitude of the foot part (12) and/or of the upper part (11) is determined by means of an inertial measurement unit (30), and wherein the resistance (Am) against pivoting is modified as a function of the presence or absence of ground contact and the spatial attitude that has been determined and/or the profile that has been determined of the spatial attitude **characterized in that** a dorsal movement is enabled or prevented as a function of the measured angle between the upper part (11) and the foot part (12) and/or a spatial attitude angle.

2. The method as claimed in claim 1, **characterized in that** the presence or absence of ground contact is determined by means of a ground reaction force, the ground reaction force being determined by means of at least one force sensor (60) on the foot part (12), on the upper part (11) and/or on the proximal prosthesis component (20).

3. The method as claimed in claim 1 or 2, **characterized in that** the presence or absence of ground contact is determined by means of at least one acceleration sensor (60) on the foot part (12), on the upper part (11) and/or on the proximal prosthesis component (20).

4. The method as claimed in one of the preceding claims, **characterized in that** a status of the foot part (12) relative to the upper part (11) and/or a pivoting movement of the foot part (12) with respect to the upper part (11) is determined, and the resistance against pivoting is modified as a function of the status and/or pivoting movement that has been determined.

5. The method as claimed in one of the preceding claims, **characterized in that** the ground reaction force is determined by means of at least one contact switch, by means of a deformation of a resilient element, by means of the recording of a rotational and/or translational relative position of two components connected flexibly to one another, by means of a pressure measurement film and/or by means of a strain gauge (DMS) as the sensor (60).

6. The method as claimed in one of the preceding claims, **characterized in that** the magnitude of the ground reaction force is measured, and the pivoting resistance is reduced or increased, or pivoting is blocked, when a threshold value of the ground reaction force is reached or exceeded.

7. The method as claimed in claim 6, **characterized in that** the threshold value is set to between 5% and 50% of the body weight of the patient.

8. The method as claimed in one of the preceding claims, **characterized in that** the angle between the foot part (12) and the upper part (11) or the proximal prosthesis component (20) is measured, and the measured angle or angle profile and the spatial attitude that has been determined of the upper part (11) or of the proximal prosthesis component (20) are used in order to modify the resistance.

9. The method as claimed in claim 1, **characterized in that** the maximum dorsal flexion angle between the upper part (11) and the foot part (12) is adjusted as a function of the ground surface inclination and/or heel height.

10. The method as claimed in claim 9, **characterized in that** the adjustment of a dorsal flexion stop is carried out through a plurality of substeps up to the maximum dorsal flexion angle.

11. The method as claimed in one of claims 8 to 10, **characterized in that** a further dorsal flexion is prevented when a limit value is reached for an inclination of the upper part (11) or of the proximal prosthesis component (20), and a further dorsal flexion is prevented when a limit value is reached for the angle between the upper part (11) and the foot part (12), the criterion occurring earlier being dominant.

12. The method as claimed in one of the preceding claims, **characterized in that** during standing, a dorsal flexion is locked when reaching a defined threshold value of the spatial orientation of the upper part (11) or of the proximal prosthesis component (20).

13. The method as claimed in claim 12, **characterized in that** the locking of the dorsal flexion is released in the event of an increase in a ground reaction force to more than 50% of the patient's weight, in the event of a change in a rotation rate of the upper part (11) and/or occurrence of an angular acceleration of the upper part (11).

14. The method as claimed in one of the preceding claims, **characterized in that** when walking on a downward sloping ground surface, a dorsal flexion stop is adjusted in the direction of an increased dorsal flexion angle.

15. The method as claimed in claim 14, **characterized in that** when walking on a downward sloping ground surface, the dorsal flexion stop is increased by from 100% to 500% compared with walking on the flat.

16. The method as claimed in one of the preceding claims, **characterized in that** the foot part (12) is moved back into an initial status in a swing phase.

17. The method as claimed in one of the preceding claims, **characterized in that** the resistance (Am) of the resistance device (40) is set to a minimum value in the event of a vertical orientation of the upper part (11).

## Revendications

1. Procédé de commande d'un pied prothétique (10) qui comprend une partie supérieure (11), munie d'un élément de fixation (13) pour un composant prothétique proximal (20), et une partie de pied (12) montée sur celle-ci de manière à pouvoir pivoter autour d'un axe de pivotement (15) et munie d'un dispositif de résistance (40) par lequel une résistance réglable (Am) est opposée à un mouvement de pivotement de la partie de pied (12) par rapport à la partie supérieure (11), et munie d'un dispositif de commande (70) qui est couplé au dispositif de résistance (40) et à au moins un capteur (60) et par lequel la résistance à un pivotement est réglée en fonction de données de capteur,
dans lequel
un contact au sol de la partie de pied (12) avec le sol est déterminé par au moins un capteur (60),
l'orientation spatiale de la partie de pied (12) et/ou de la partie supérieure (11) est déterminée par une unité de mesure inertielle (30), et
la résistance (Am) à un pivotement est modifiée en fonction de la présence ou de l'absence d'un contact au sol et en fonction de l'orientation spatiale déterminée et/ou de l'évolution déterminée de l'orientation spatiale,
**caractérisé en ce qu'**un mouvement dorsal est autorisé ou empêché en fonction de l'angle mesuré entre la partie supérieure (11) et la partie de pied (12) et/ou d'un angle de l'orientation spatiale.

2. Procédé selon la revendication 1,
**caractérisé en ce que** la présence ou l'absence d'un contact au sol est déterminée par une force de réaction au sol, la force de réaction au sol étant déterminée par au moins un capteur de force (60) situé sur la partie de pied (12), sur la partie supérieure (11) et/ou sur le composant prothétique proximal (20).

3. Procédé selon la revendication 1 ou 2,
**caractérisé en ce que** la présence ou l'absence d'un contact au sol est déterminée par au moins un capteur d'accélération (60) situé sur la partie de pied (12), sur la partie supérieure (11) et/ou sur le composant prothétique proximal (20).

4. Procédé selon l'une des revendications précédentes,
**caractérisé en ce qu'**une position de la partie de pied (12) par rapport à la partie supérieure (11) et/ou un mouvement de pivotement de la partie de pied (12) par rapport à la partie supérieure (11) sont déterminés et la résistance à un pivotement est modifiée en fonction de la position et/ou du mouvement de pivotement déterminés.

5. Procédé selon l'une des revendications précédentes,
**caractérisé en ce que** la force de réaction au sol est déterminée par au moins un interrupteur de contact, par une déformation d'un élément élastique, par la détection d'une position relative en rotation et/ou en translation de deux composants reliés de manière flexible l'un à l'autre, par une feuille de mesure de pression et/ou par une jauge de contrainte, servant de capteur (60).

6. Procédé selon l'une des revendications précédentes,
**caractérisé en ce que** la hauteur de la force de réaction au sol est mesurée et que, lorsqu'une valeur seuil de la force de réaction au sol est atteinte ou dépassée, la résistance au pivotement est diminuée ou augmentée ou un pivotement est bloqué.

7. Procédé selon la revendication 6,
**caractérisé en ce que** la valeur seuil est réglée pour prendre une valeur entre 5 % et 50 % du poids du corps du patient.

8. Procédé selon l'une des revendications précédentes,
**caractérisé en ce que** l'angle entre la partie de pied (12) et la partie supérieure (11) ou le composant prothétique proximal (20) est mesuré, et l'angle ou le tracé angulaire mesuré et l'orientation spatiale déterminée de la partie supérieure (11) ou du composant prothétique proximal (20) sont utilisés pour modifier la résistance.

9. Procédé selon la revendication 1,
**caractérisé en ce que** l'angle maximal de flexion dorsale entre la partie supérieure (11) et la partie de pied (12) est réglé en fonction de l'inclinaison du sol et/ou de la hauteur du talon.

10. Procédé selon la revendication 9,
**caractérisé en ce que** le réglage d'une butée de flexion dorsale s'effectue en plusieurs étapes partielles jusqu'à atteindre l'angle maximal de flexion dorsale.

11. Procédé selon l'une des revendications 8 à 10,
**caractérisé en ce que**, lorsqu'une valeur limite est atteinte pour une inclinaison de la partie supérieure (11) ou du composant prothétique proximal (20), une flexion dorsale supplémentaire est empêchée et, dès qu'une valeur limite de l'angle entre la partie supérieure (11) et la partie de pied (12) est atteinte, une flexion dorsale supplémentaire est empêchée, le critère qui intervient en premier étant déterminant.

12. Procédé selon l'une des revendications précédentes,
**caractérisé en ce que**, en position debout, une flexion dorsale est bloquée lorsqu'une valeur seuil fixée de l'orientation spatiale de la partie supérieure (11) ou du composant prothétique proximal (20) est atteinte.

13. Procédé selon la revendication 12,
**caractérisé en ce que** le blocage de la flexion dorsale est supprimé en cas d'augmentation d'une force de réaction au sol à plus de 50 % du poids du patient, en cas de modification d'une vitesse de rotation de la partie supérieure (11) et/ou en cas d'apparition d'une accélération angulaire de la partie supérieure (11).

14. Procédé selon l'une des revendications précédentes,
**caractérisé en ce que**, lors de la marche sur un sol en descente, une butée de flexion dorsale est réglée dans le sens d'un angle accru de flexion dorsale.

15. Procédé selon la revendication 14,
**caractérisé en ce que**, lors de la marche sur un sol en descente, la butée de flexion dorsale est augmentée de 100 % à 500 % par rapport à la marche sur le plat.

16. Procédé selon l'une des revendications précédentes,
**caractérisé en ce que** la partie de pied (12) est ramenée dans une position initiale lors d'une phase d'oscillation.

17. Procédé selon l'une des revendications précédentes,
**caractérisé en ce que**, lorsque la partie supérieure (11) est orientée verticalement, la résistance (Am) du dispositif de résistance (40) est réglée sur une valeur minimale.
